# EUROPEAN PATENT SPECIFICATION

(11) **EP 1 632 233 B1**
(45) Date of publication and mention of the grant of the patent: **02.07.2008**
(21) Application number: 04733089.9
(22) Date of filing: 14.05.2004
(51) Int. Cl.: A61K 31/51, A61K 31/7004, A61K 33/14, A61K 31/405, A61K 31/4172, A61K 31/401, A61K 31/198, A61P 3/02

(54) **TRANSFUSION PREPARATION FOR PERIPHERAL INTRAVENOUS ADMINISTRATION AND METHOD OF STABILIZING VITAMIN B1**
TRANSFUSIONSZUBEREITUNG ZUR PERIPHEREN INTRAVENÖSEN VERABREICHUNG UND VERFAHREN ZUR STABILISIERUNG VON VITAMIN B1
PREPARATION DE TRANSFUSIONS POUR ADMINISTRATION INTRAVEINEUSE PERIPHERIQUE ET PROCEDE DE STABILISATION DE LA VITAMINE B1

(30) Priority: 22.05.2003 JP 2003145353
(43) Date of publication of application: 08.03.2006
(73) Proprietor: OTSUKA PHARMACEUTICAL FACTORY, INC., Naruto-shi, Tokushima 772-8601 (JP)
(72) Inventor: MITSUMOTO, Yasuhiro, Otsu-cho Naruto-shi, Tokushima 772-0031 (JP); ARITA, Shigeaki;, Itano-gun Tokushima;7710204 (JP); TANI, Seiji;, Tokushima;7700901 (JP); SUMIYOSHI, Nobuaki;, Naruto-shi Tokushima;7710360 (JP)
(74) Representative: W.P. Thompson & Co.
(86) International application number: PCT/JP2004/006539
(87) International publication number: WO 2004/103375

(56) References cited:
- EP-A- 0 712 583
- JP-A- 6 312 923
- JP-A- 8 143 459
- JP-A- 9 059 150
- JP-A- 10 087 497
- JP-A- 10 087 498
- JP-A- 11 035 471
- JP-A- 2003 055 195
- JP-B2- 3 028 403
- DATABASE WPI Section Ch, Week 200608 Derwent Publications Ltd., London, GB; Class B05, AN 2006-072496 XP002375464 -& JP 2006 001945 A (TERUMO CORP) 5 January 2006 (2006-01-05)
- PATENT ABSTRACTS OF JAPAN vol. 1999, no. 11, 30 September 1999 (1999-09-30) -& JP 11 158061 A (OTSUKA PHARMACEUT FACTORY INC), 15 June 1999 (1999-06-15)
- DATABASE WPI Section Ch, Week 200165 Derwent Publications Ltd., London, GB; Class B05, AN 2001-574437 XP002375466 -& JP 2001 163780 A (TANABE SEIYAKU CO) 19 June 2001 (2001-06-19)
- DATABASE WPI Section Ch, Week 200104 Derwent Publications Ltd., London, GB; Class B05, AN 2001-027429 XP002375467 -& JP 2000 273035 A (TANABE SEIYAKU CO) 3 October 2000 (2000-10-03)

## Description

### Technical Field

The present invention relates to an infusion preparation for peripheral parenteral administration that stably contains vitamin B1 and to a method for stabilizing vitamin B1.

### Background Art

Conventionally, in order to supply all the nutrients necessary for life support to patients who have difficulties in receiving nourishment per oral, infusion therapy to administer an infusion through veins has been widely carried out. Nutrients to be administered include not only sugars, amino acids, electrolytes, but also minerals, vitamins and other nutrients necessary for life support.

It is well known that when a high-calorie infusion is administered through central veins (TPN, IVH), vitamin B1 deficiency inhibits aerobic glycolysis, thus producing lactic acid and causing severe lactic acidosis. Adding vitamin B1 is essential. For this reason, a high-calorie infusion preparation that has vitamin B1 added in advance has been under consideration (see Patent documents 1 to 4 below).

On the other hand, it is reported that even when a medium-calorie general infusion of nutrient is administered through peripheral veins during a relatively short period (PPN), vitamin B 1 deficiency can occur by lowering vitamin B 1 concentration in blood, though it is not so severe as to administer a high-calorie infusion (see Nonpatent document 1 below). Attempts have been made to add vitamin B1 also to an infusion for peripheral parenteral administration in advance (see Patent document 5).

Vitamin B1 solution is unstable between neutrality and alkaline, and is decomposed by sulfite ions. Therefore, by giving a specific pH to an infusion containing vitamin solution and adding no sulfite or minimum sulfite, vitamin B1 in the above infusion preparations has been stabilized.

In the above conventional infusion preparations, the infusion to which vitamin B 1 is added has a specific characteristic, so that vitamin B 1 can keep stabilized. Even so, there is still demand for further stabilized vitamin B1.
Patent document 1: Japanese Patent Publication Laid-Open No. 8-143459
Patent document 2: Japanese Patent Publication Laid-Open No. 9-59150
Patent document 3: Japanese Patent Publication Laid-Open No. 10-226636
Patent document 4: Japanese Patent Publication Laid-Open No. 11-35471
Patent document 5: Japanese Patent Publication Laid-Open No. 2003-55195
Nonpatent document 1: Nakamura et al., "Thiamine Deficiency in Critically III Patients under Peripheral Parenteral Nutrition", The Japanese Journal of Surgical Metabolism and Nutrition. 36 (6), 307 (2002)
JP11158061 discloses an infusion solution for central vein administration; the infusion comprising (A) a solution containing reducing sugar, (B) a solution containing amino acids and (C) a solution containing lipophilic vitamins.
JP2001163780 discloses a total nutrition infusion solution comprising transfusion liquids filled in separate chambers of a container connected with separation units. The first solution comprises amino acids and vitamin C at pH 5 to 8 and the second solution comprises reducing sugar, vitamin B1, B2 and L-tyrosine at pH 2.5 to 5.

### Disclosure of the Invention

### Problem to be Solved by the Invention

The advantage of the present invention is to provide an infusion preparation for peripheral parenteral administration that contains much more stable vitamin B 1 without losing safety and efficacy as a preparation, and a method for stabilizing vitamin B1.

### Means for solving the problem

After keen examinations to solve the above problem, the inventors of the present invention have found that buffering salt contained in an infusion prevents vitamin B 1 from being stabilized. The inventors have achieved the present invention, finding the new fact that when electrolytes in a vitamin B1-containing infusion have titratable acidity of one or less, a preparation that contains much more stable vitamin B1 without losing safety and efficacy can be attained.

The present invention therefore provides an infusion preparation for peripheral parenteral administration, comprising an infusion (A) containing glucose with 80 to 200 g/L concentration and an infusion (B) containing amino acid with 50 to 150 g/L concentration that are separately put into a vessel that is divided with an openable partition means;
wherein the infusion (A) does not contain sulfite, has titratable acidity of 1 or less, contains vitamin B1, is adjusted to pH 3 to 5, contains carboxylic acid and its salt with 0 to 5mEq/L concentration, and wherein further electrolytes contained in the infusion (A) are all strong electrolytes;
the infusion (B) is adjusted to pH 6.5 to 8;
after mixing the infusion (A) and the infusion (B), the mixture has pH 6 to 7.5 and titratable acidity of 5 to 10;
and infusion (A) has a potassium concentration of 10 to 20 mEq/L and infusion (B) has a potassium concentration of 20 to 40 mEq/L.
The infusion (A) of the present invention may have electrolyte composition as follows: Ca²⁺: 2 to 10 mEq/L, Mg²⁺: 2 to 10 niEq/L, Cl⁻: 12 to 30 mEq/L, Zn: 2 to 10 mmol/L. The infusion (B) may have electrolyte composition as follows: Na⁺: 80 to 150 mEq/L, P: 10 to 20 mmol/L. The volume ratio (A:B) of the infusion (A) and the infusion (B) may be 1 to 4:1.

In particular, in the present invention, it is preferable that calcium and potassium are respectively contained in the form of chloride as the above strong electrolytes, and that sodium chloride is not used as a sodium supply source in either of the infusions (A) or (B).

Moreover, preferably, the vessel is a flexible plastic infusion bag having at least two chambers that are separated by an easily removable seal.

The present invention also provides a method for stabilizing vitamin B 1 in an infusion preparation for peripheral parenteral administration which comprises an infusion (A) containing vitamin B1 and 80 to 200 g/L of glucose and an infusion (B) containing 50 to 150 g/L of amino acid and being adjusted to pH 6.5 to 8 that are separately put into a vessel that is divided with an openable partition means and wherein after mixing the infusion (A) and the infusion (B), the mixture has pH 6 to 7.5 and titratable acidity of 5 to 10,
wherein the infusion (A) does not contain sulfite, has titratable acidity of 1 or less, is adjusted to pH 3 to 5, contains carboxylic acid and its salt with 0 to 5 mEq/L concentration, and
wherein further electrolytes contained in the infusion (A) are all strong electrolytes,
and infusion A has a potassium concentration of 10 to 20 mEq/L and infusion (B) has a potassium concentration of 20 to 40 mEq/L.

### Effect of the Invention

The infusion preparation for peripheral parenteral administration in the present invention has an effect of further improving stability of vitamin B 1 without losing safety and efficacy.

### Preferred Embodiments for Practicing the Invention

The infusion preparation for peripheral parenteral administration (hereafter, referred to simply as infusion preparation) in the present invention will be now described in detail. In the infusion preparation of the present invention, the infusion (A) containing glucose and the infusion (B) containing amino acid are separately put into a vessel that is divided with an openable partition means, and when used, the infusions (A) and (B) are mixed.

### <Infusion (A)>

In the present invention, the infusion (A) is basically composed of glucose, strong electrolytes and vitamin B1. To stabilize vitamin B1, the infusion (A) does not contain sulfite. Glucose is used with 80 to 200 g/L concentration, preferably, with 80 to 150 g/L concentration. It is preferable that pH in the glucose solution is adjusted with mineral acid such as hydrochloric acid to eliminate buffer property as much as possible. The pH range of the infusion (A) is 3 to 5, preferably, 3.5 to 4.5. The infusion (A) at pH of not more than 3 can be excellent in stabilizing vitamin B1 while allowing glucose to be unstable. On the other hand, the infusion (A) at pH of not less than 5 allows vitamin B 1 to lose stability.

Preferably, the glucose solution has fluid volume of 200 to 1000mL. Distilled water for injection is normally used as a solvent of the glucose solution. In addition, to further improve stability of vitamin B1 and make it easy to adjust the pH of the mixture described later to 6 to 7.5, the glucose solution has titratable acidity of 1 or less, preferably 0.5 or less, more preferably 0.1 or less.

Besides glucose, one or more of reducing sugars such as fructose, maltose etc., or nonreducing sugars such as sorbitol, glycerin etc. may be contained in moderation.

### <Infusion (B)>

In the present invention, the infusion (B) is an amino acid solution, and contains amino acid composition composed of at least essential amino acid. Amino acid is contained with 50 to 150 g/L concentration, preferably, with 80 to 120 g/L concentration, in terms of free amino acid. Each amino acid used is preferably purely crystalline amino acid as in a general amino acid infusion. Though these amino acids are normally used in the form of free amino acid, they can be used not only in such a form, but also in the form of pharmacologically accepted salt, ester, N-acyl derivative, salt of two amino acids and peptide. Especially, it is suitable that L-cysteine is contained as N-acetyl compound in terms of stability. Fluid volume of the infusion (B) to be put into an infusion vessel may be 100 to 500mL. Distilled water for injection is normally used as a solvent of the infusion (B).

Preferable composition of amino acid is shown as follows in terms of free amino acid: L-leucine: 10 to 20 (g/L), L-isoleucine: 5 to 15 (g/L), L-valine: 5 to 15 (g/L), L-lysine: 5 to 15 (g/L), L-threonine: 2 to 10 (g/L), L-tryptophan: 0.5 to 5 (g/L), L-methionine: 1 to 8 (g/L), L-phenylalanine: 3 to 15 (g/L), L-cysteine: 0.1 to 3 (g/L), L-tyrosine: 0.1 to 2 (g/L), L-arginine: 5 to 15 (g/L), L-histidine: 2 to 10 (g/L), L-alanine: 5 to 15 (g/L), L-proline: 2 to 10 (g/L), L-serine: 1 to 7 (g/L), Glycine: 2 to 10 (g/L), L-asparatic acid: 0.2 to 3 (g/L), L-glutamic acid: 0.2 to 3 (g/L).

By adding a small amount of pH adjuster when necessary, the infusion (B) is adjusted to pH 6.5 to 8.0, preferably, to pH 6.7 to 7.5. If pH in the infusion (B) is below 6.5, the mixture of the infusions cannot keep its pH within the optimal range as described later. To the contrary, if pH in the infusion (B) is over 8.0, easily oxidizable amino acids including L-cysteine etc. become more unstable. The both cases are not preferable.

### <Vitamin B1>

Vitamin B1 is contained in the infusion (A) as thiamine with 1 to 10 mg/L concentration, preferably, with 2 to 5 mg/L concentration. It is suitable to contain 0.5 to 8 mg of vitamin B1 as absolute amount. As vitamin B1 (thiamine), thiamine hydrochloride, thiamine nitrate, prosultiamine and octotiamine etc. can be used.

### (Electrolyte)

### (a) Potassium

Potassium is contained in the infusion (A) and the infusion (B) separately. As for concentration of potassium contained in each infusion the infusion (A) has potassium concentration of 10 to 20 mEq/L and the infusion (B) has potassium concentration of 20 to 40 mEq/L. Potassium contained in the infusion (A) and the infusion (B) is preferably 5 to 30 mEq in total.

As a potassium supply source contained in the infusion (A), potassium chloride, potassium sulfate and the like that are strong electrolytes are preferable. In particular, potassium chloride is more preferable since it is generally used. On the other hand, as a potassium supply source contained in the infusion (B), such compounds as are used in general electrolyte infusions can be used. Potassium chloride, potassium acetate, potassium citrate, potassium dihydrogen phosphate, dipotassium hydrogen phosphate, potassium glycerophosphate, potassium sulfate and potassium lactate can be cited as examples. Among these, phosphate such as potassium dihydrogen phosphate, dipotassium hydrogen phosphate and potassium glycerophosphate is suitable as a phosphorus supply source as well. The potassium supply source may be in hydrated form.

### (b) Calcium

It is preferable that calcium is contained only in the infusion (A). If calcium is contained in the infusion (B), precipitation occurs, reacting to phosphate. Prevention of such precipitation is the reason why calcium and phosphate are separated. Calcium chloride that is a strong electrolyte is preferably used as a calcium supply source. It is also preferable that calcium is contained with 2 to 10 mEq/L concentration in the infusion (A).

### (c) Sodium

Sodium can be contained in either or both of the infusion (A) and the infusion (B). However, it is preferable to use chloride for potassium and calcium. Therefore, it is preferable that sodium chloride is not used as a sodium supply source to prevent hyperchloremic acidosis from occurring.

When buffering sodium salts such as sodium acetate, sodium citrate, sodium dihydrogen phosphate, disodium hydrogen phosphate and sodium lactate are used, it is preferable that such sodium salts are added to the infusion (B) so as to meet the above-mentioned requirement for titratable acidity in the infusion (A). It is also preferable that sodium is contained with 80 to 150 mEq/L concentration in the infusion (B).

To prevent phosphorus and calcium or magnesium from being precipitated after mixing the infusions, sodium citrate is suitably used as part of sodium supply source.

### (d) Other electrolytes

(i) Examples of magnesium supply source include magnesium sulfate, magnesium chloride, magnesium acetate and the like. Among these, magnesium sulfate and magnesium chloride can be contained in the infusion (A) as strong electrolytes.
(ii) Examples of phosphorus supply source include potassium dihydrogen phosphate, dipotassium hydrogen phosphate, potassium glycerophosphate, sodium dihydrogen phosphate, disodium hydrogen phosphate, sodium glycerophosphate and the like. These phosphorus compounds are contained in the infusion (B).
(iii) Examples of zinc supply source include zinc sulfate, zinc chloride and the like. These zinc compounds can be contained in the infusion (A).

As a supply source of each electrolyte mentioned in (i) to (iii), hydrate can be used. However, buffering electrolytes should be added to the infusion (B). Regarding each electrolyte, it is preferable that the infusion (A) contains magnesium with 2 to 10 mEq/L concentration and zinc with approximately 2 to 10 mmol/L concentration. It is also preferable that the infusion (B) contains phosphorus with approximately 10 to 20 mmol/L concentration.

### <Additive, Combination drug>

Additives such as stabilizers can be added to the infusion preparation of the present invention if necessary. However, it is suitable that sulfite such as sodium hydrogen sulfite typical of stabilizers is added to the infusion (B). In addition, if necessary, other combination drugs including each type of vitamins, trace elements (minerals) and the like can be optionally added to and contained in the infusion preparation of the present invention.

### (Mixture)

In the infusion preparation of the present invention, the infusion (A) and the infusion (B) are mixed when used To prevent patients from suffering angialgia and increase safety, the mixture of the infusions (A) and (B) preferably has pH 6 to 7.5 and titratable acidity of 5 to 10. The volume ratio of the infusion (A) and the infusion (B) is preferably 1 to 4:1.

### <Infusion solution vessel>

A vessel for putting the infusion preparation of the present invention into is not particularly limited, as long as it has two chambers that can be communicated with each other. As examples of a vessel (infusion bag) having two chambers that are separated by an openable partition, the followings can be cited: the vessel which has a partition formed with an easily removable seal (Japanese Patent Publication Laid-Open No. 2-4671, Japanese Utility Model Publication Laid-Open No. 5-5138 etc.), the vessel which has a partition formed by placing a clip between the chambers (Japanese Patent Publication Laid-Open No. 63-309263 etc.), and the vessel which has various openable means prepared for a partition. (Japanese Examined Patent Application Publication No. 63-20550 etc.). Among these, an infusion bag that has a partition formed with an easily removable seal is suitable for mass production and preferable in terms of easy communication.

Examples of materials of the above infusion bag include flexible plastics such as various gas-permeable plastics including polyethylene, polypropylene, polyvinylchloride, cross-linked ethylene-vinyl acetate copolymer, ethylene-alpha-olefin copolymer, and a blend or a laminated body of these respective polymers, each of which is widely used for medical packaging.

The infusion preparation of the present invention can be put into or fill an infusion bag by conventional methods. For example, there is a method of filling each chamber with each infusion in an inert gas atmosphere, then putting a stopper and carrying out heat sterilization. Well-known methods such as high-pressure steam sterilization and hot water shower sterilization can be applied for the heat sterilization. If necessary, heat sterilization can be carried out in an inert gas atmosphere such as carbon dioxide and nitrogen.

Moreover, to surely protect the infusion preparation put into the above infusion bag against deterioration and oxidation, preferably, the infusion bag and a deoxidizer are packed together with an oxygen-barrier outer packaging bag. In particular, when a two-chambered infusion bag that has a partition formed with an easily removable seal is used, it is preferable that the infusion bag is packed, being folded at the portion of an easily removable seal, for example, being folded in two at the portion of an easily removable seal, so that the partition cannot be open due to outside pressure. In addition, if necessary, inert gas filled packaging and the like can be applied.

As materials of a gas-impermeable outer packaging vessel suitable for the above packaging, films and sheets that are made of various materials and generally used can be employed. Specifically, ethylene-vinylalcohol copolymer, polyvinylidene chloride, polyacrylonitrile, polyvinyl alcohol, polyamide, polyester etc. or a material containing at least one of these can be cited as examples.

Regarding a deoxidizer, each type of well-known deoxidizers, specifically, deoxidizers containing iron compounds such as iron hydroxide, iron oxide and iron carbide as an active ingredient or deoxidizers containing low-molecular phenol and active carbon can be used. As typical commercial items, "Ageless" (by Mitsubishi Gas Chemical Co.), "Modulan" (by Nippon Kayaku Co.), "Secule" (by Nippon Soda Co.) and "Tamotsu" (by Oji Kako Co.) can be named,

The present invention will be described in detail below, referring to examples and comparative examples.

### EXAMPLE 1

### <Infusion (A)>

Glucose and each strong electrolyte were dissolved in distilled water for injection with the following concentration to prepare the infusion (A) having the composition as mentioned below. In this infusion (A), a small amount of hydrochloric acid was added to adjust pH to 4.5. The titratable acidity of the infusion (A) was 0.08.

| | |
|---|---|
| Glucose | 107.14 g/L |
| Potassium chloride | 0.92 g/L |
| Calcium chloride (2H₂O) | 0.53 g/L |
| Magnesium sulfate (7H₂O) | 0.88 g/L |
| Zinc sulfate | 2.00 mg/L |
| Thiamine hydrochloride | 2.71 mg/L |

### <Infusion (B)>

The following crystalline amino acids and each electrolyte were dissolved in distilled water for injection to prepare the infusion (B) having the composition as mentioned below. In this infusion (B), acetic acid was used as pH adjuster to adjust pH to 6.8.

| | |
|---|---|
| L-leucine | 14.0 g/L |
| L-isoleucine | 8.0 g/L |
| L-valine | 8.0 g/L |
| L-lysine hydrochloride | 13.1 g/L |
| L-threonine | 5.7 g/L |
| L-tryptophan | 2.0 g/L |
| L-methionine | 3.9 g/L |
| L-phenylalanine | 7.0 g/L |
| N-acetyl-L-cysteine | 1.3 g/L |
| L-tyrosine | 0.5 g/L |
| L-arginine | 10.5 g/L |
| L-histidine | 5.0 g/L |
| L-alanine | 8.0 g/L |
| L-proline | 5.0 g/L |
| L-serine | 3.0 g/L |
| Glycine | 5.9 g/L |
| L-asparatic acid | 1.0 g/L |
| L-glutamic acid | 1.0 g/L |
| Dipotassium hydrogen phosphate | 3.31 g/L |
| Disodium hydrogen phosphate | 5.13 g/L |
| Sodium lactate | 7.63 g/L |
| Sodium citrate | 1.77 g/L |
| Sodium hydrogen sulfite | 0.05 g/L |

### <Infusion preparation>

The both infusions obtained as above went through aseptic filtration. The infusion (A) 700 mL and the infusion (B) 300mL were respectively put into each chamber of a polyethylene vessel that has two chambers separated by an easily removable seal. The infusion (B) went through nitrogen replacement and was closely sealed. Then, high-pressure steam sterilization was performed according to a conventional method. Subsequently, the vessel was folded at the portion of an easily removable seal and was put into an outer packaging bag (oxygen-barrier outer packaging bag) made of multilayer barrier film (Product name: "Bovlon" by NSR), together with a deoxidizer (Product name: "Ageless" by Mitsubishi Gas Chemical Co.). Thereby, the infusion preparation of the present invention was obtained. After mixing the two infusions constituting this infusion preparation, the mixture had pH 6.7 and titratable acidity of 7.

### EXAMPLE 2

Except that instead of hydrochloric acid in the infusion (A) of Example 1, acetic acid was used to adjust pH to 4.5, an infusion preparation was obtained in a similar way to Example 1. The titratable acidity of the infusion (A) was 0.1, and the concentration of acetic acid was 0.2 mEq/L. After mixing two infusions, the mixture had pH 6.7 and titratable acidity of 7.

### EXAMPLE 3

Except that instead of 0.88 g/L of magnesium sulfate (7H₂O) in the infusion (A) of Example 2, 0.44 g/L of magnesium sulfate (7H₂O) and 0.38 g/L of magnesium acetate (4H₂O) were added, an infusion preparation was obtained in a similar way to Example 2. The titratable acidity of the infusion (A) was 0.5, and the concentration of acetic acid was 4.4 mEq/L. After mixing two infusions, the mixture had pH 6.6 and titratable acidity of 7.5.

### Comparative Example 1

Instead of 7.63 g/L of sodium lactate added in the infusion (B) of Example 1, 3.27 g/L of sodium lactate was added to the infusion (A). Except for this, an infusion preparation was obtained in a similar way to Example 1. The titratable acidity of the infusion (A) was 4.2.

### Comparative Example 2

Instead of 0.53 g/L of calcium chloride (2H₂O) in the infusion (A) of Example 1, 1.6 g/L of calcium gluconate was added, and instead of 7.63 g/L of sodium lactate added in the infusion (B), 3.27 g/L of sodium lactate was added to the infusion (A). Except for these, an infusion preparation was obtained in a similar way to Example 1. The titratable acidity of the infusion (A) was 4.9.

### Comparative Example 3

Except that instead of magnesium sulfate (7H₂O) in the infusion (A) of Example 2, 0.77 g/L of magnesium acetate (4H₂O) was added, an infusion preparation was obtained in a similar way to Example 2. The titratable acidity of the infusion (A) was 1.6, and the concentration of acetic acid was 8.5 mEq/L. After mixing two infusions, the mixture had pH 6.6 and titratable acidity of 8. [0051] After storing the infusion preparations obtained in the above examples 1 to 3 and comparative examples 1 to 3 under the condition of 60°C and 75%RH for 14 days, the infusion bags were picked up from the outer packaging bags. The infusion (A) only was withdrawn into a syringe to measure residual volume of vitamin B1 with a high-performance liquid chromatography and figure out the residual ratio of vitamin B 1 to initially supplied amount.

**[Table 1]**

| | Example 1 | Example 2 | Example 3 | Comparative Example 1 | Comparative Example 2 | Comparative Example 3 |
|---|---|---|---|---|---|---|
| Vitamin B1 residual ratio (%) | 91.3 | 91 | 88 | 80.3 | 75.5 | 82.5 |

It is apparent from the result shown in Table 1 that compared to the infusion preparations of comparative examples 1 to 3, the infusion preparations of examples 1 to 3 have more stable vitamin B 1 contained in the infusion (A).

## Claims

1. An infusion preparation for peripheral parenteral administration, comprising an infusion (A) containing glucose with 80 to 200 g/L concentration and an infusion (B) containing amino acid with 50 to 150 g/L concentration that are separately put into a vessel that is divided with an openable partition means;
wherein the infusion (A) does not contain sulfite, has titratable acidity of 1 or less, contains vitamin B1, is adjusted to pH 3 to 5, contains carboxylic acid and its salt with 0 to 5mEq/L concentration, and wherein further electrolytes contained in the infusion (A) are all strong electrolytes;
the infusion (B) is adjusted to pH 6.5 to 8;
after mixing the infusion (A) and the infusion (B), the mixture has pH 6 to 7.5 and titratable acidity of 5 to 10;
and infusion (A) has a potassium concentration of 10 to 20 mEq/L and infusion (B) has a potassium concentration of 20 to 40 mEq/L.

2. The infusion preparation for peripheral parenteral administration according to claim 1, where in the infusion (A) has electrolyte composition of Ca²⁺: 2 to 10 mEq/L, Mg²⁺: 2 to 10 mEq/L, Cl⁻: 12 to 30 mEq/L, Zn: 2 to 10 mmol/L; the infusion (B) has electrolyte composition of Na⁺: 80 to 150 mEq/L, P: 10 to 20 mmol/L; and the volume ratio (A:B) of the infusion (A) and the infusion (B) is 1 to 4:1.

3. The infusion preparation for peripheral parenteral administration according to claim 2, wherein calcium and potassium are contained respectively in the form of chloride as the strong electrolytes; and sodium chloride is not used as a sodium supply source in either of the infusions (A) or (B).

4. The infusion preparation for peripheral parenteral administration according to claim 1, wherein amino acid composition in the infusion (B) is shown as follows in terms of free amino acid: L-leucine: 10 to 20 (g/L), L-isoleucine: 5 to 15 (g/L), L-valine: 5 to 15 (g/L), L-lysine: 5 to 15 (g/L), L-threonine: 2 to 10 (g/L), L-tryptophan: 0.5 to 5 (g/L), L-methionine: 1 to 8 (g/L), L-phenylalanine: 3 to 15 (g/L), L-cysteine: 0.1 to 3 (g/L), L-tyrosine: 0.1 to 2 (g/L), L-arginine: 5 to 15 (g/L), L-histidine: 2 to 10 (g/L), L-alanine: 5 to 15 (g/L), L-proline: 2 to 10 (g/L), L-serine: 1 to 7 (g/L), Glycine: 2 to 10 (g/L), L-aspartic acid: 0.2 to 3 (g/L), L-glutamic acid: 0.2 to 3 (g/L).

5. The infusion preparation for peripheral parenteral administration according to claim 4, wherein L-cysteine is contained as N-acetyl compound.

6. The infusion preparation for peripheral parenteral administration according to claim 5, wherein vitamin B1 is contained in the infusion (A) with 1 to 10 mg/L concentration in tenns of thiamine.

7. The infusion preparation for peripheral parenteral administration according to claim 1, wherein the vessel is a flexible plastic infusion bag having at least two chambers that are separated by an easily-removable seal.

8. The infusion preparation for peripheral parenteral administration according to claim 7, wherein the infusion bag was folded at the portion of the easily removable seal and was put into an oxygen-barrier outer packaging bag, together with a deoxidizer.

9. The infusion preparation for peripheral parenteral administration according to claim 1, which goes through heat sterilization.

10. A method for stabilizing vitamin B 1 in an infusion preparation for peripheral parenteral administration which comprises an infusion (A) containing vitamin B 1 and 80 to 200 g/L of glucose and an infusion (B) containing 50 to 150 g/L of amino acid and being adjusted to pH 6.5 to 8 that are separately put into a vessel that is divided with an openable partition means and wherein after mixing the infusion (A) and the infusion (B), the mixture has pH 6 to 7.5 and titratable acidity of 5 to 10,
wherein the infusion (A) does not contain sulfite, has titratable acidity of 1 or less, is adjusted to pH 3 to 5, contains carboxylic acid and its salt with 0 to 5 mEq/L concentration, and wherein further electrolytes contained in the infusion (A) are all strong electrolytes,
and infusion A has a potassium concentration of 10 to 20 mEq/L and infusion (B) has a potassium concentration of 20 to 40 mEq/L.

## Patentansprüche

1. Infusionszubereitung zur peripheren parenteralen Verabreichung, umfassend eine Infusion (A), die Glucose in einer Konzentration von 80 bis 200 g/L enthält und eine Infusion (B), die Aminosäure in einer Konzentration von 50 bis 150 g/L enthält, die separat in einen Behälter gegeben werden, der mit einem zu öffnenden Abtrennmittel unterteilt ist;
wobei die Infusion (A) kein Sulfit enthält, einen titrierbaren Säuregehalt von 1 oder weniger aufweist, Vitamin B1 enthält, auf einen pH-Wert von 3 bis 5 eingestellt ist, Carbonsäure und ihr Salz in einer Konzentration von 0 bis 5 mEq/L enthält und wobei alle weiteren Elektrolyte, die in der Infusion (A) enthalten sind, starke Elektrolyte sind; die Infusion (B) auf einen pH-Wert von 6,5 bis 8 eingestellt ist;
die Mischung nach dem Mischen der Infusion (A) und der Infusion (B) einen pH-Wert von 6 bis 7,5 und einen titrierbaren Säuregehalt von 5 bis 10 aufweist;
und Infusion (A) eine Kaliumkonzentration von 10 bis 20 mEq/L und Infusion (B) eine Kaliumkonzentration von 20 bis 40 mEq/L aufweist.

2. Infusionszubereitung zur peripheren parenteralen Verabreichung nach Anspruch 1, wobei die Infusion (A) eine Elektrolytzusammensetzung von Ca²⁺: 2 bis 10 mEq/L, Mg²⁺: 2 bis 10 mEq/L, Cl⁻: 12 bis 30 mEq/L, Zn: 2 bis 10 mmol/L aufweist; die Infusion (B) eine Elektrolytzusammensetzung von Na⁺: 80 bis 150 mEq/L, P: 10 bis 20 mmol/L aufweist; und das Volumenverhältnis (A:B) der Infusion (A) und der Infusion (B) 1 bis 4:1 beträgt.

3. Infusionszubereitung zur peripheren parenteralen Verabreichung nach Anspruch 2, wobei Calcium und Kalium jeweils in Form ihrer Chloride als starke Elektrolyte enthalten sind; und Natriumchlorid weder in Infusion (A) noch(B) als Natriumversorgungsquelle verwendet wird.

4. Infusionszubereitung zur peripheren parenteralen Verabreichung nach Anspruch 1, wobei die Aminosäurezusammensetzung in der Infusion (B) wie folgt in Form der freien Aminosäure dargestellt ist: L-Leucin: 10 bis 20 (g/L), L-Isoleucin: 5 bis 15 (g/L), L-Valin: 5 bis 15 (g/L), L-Lysin: 5 bis 15 (g/L), L-Threonin: 2 bis 10 (g/L), L-Tryptophan: 0,5 bis 5 (g/L), L-Methionin: 1 bis 8 (g/L), L-Phenylalanin: 3 bis 15 (g/L), L-Cystein: 0,1 bis 3 (g/L), L-Tyrosin: 0,1 bis 2 (g/L), L-Arginin: 5 bis 15 (g/L), L-Histidin: 2 bis 10 (g/L), L-Alanin: 5 bis 15 (g/L), L-Prolin: 2 bis 10 (g/L), L-Serin: 1 bis 7 (g/L), Glycin: 2 bis 10 (g/L), L-Asparaginsäure: 0,2 bis 3 (g/L), L-Glutaminsäure: 0,2 bis 3 (g/L).

5. Infusionszubereitung zur peripheren parenteralen Verabreichung nach Anspruch 4, wobei L-Cystein als N-Acetylverbindung enthalten ist.

6. Infusionszubereitung zur peripheren parenteralen Verabreichung nach Anspruch 5, wobei Vitamin B1 in der Infusion (A) in einer Konzentration von 1 bis 10 mg/L in Form von Thiamin enthalten ist.

7. Infusionszubereitung zur peripheren parenteralen Verabreichung nach Anspruch 1, wobei der Behälter ein flexibler Kunststoffinfusionsbeutel ist, der mindestens zwei Kammern aufweist, die durch einen leicht entfernbaren Verschluss getrennt sind.

8. Infusionszubereitung zur peripheren parenteralen Verabreichung nach Anspruch 7, wobei der Infusionsbeutel in dem Bereich des leicht entfernbaren Verschlusses gefaltet wurde und in einen äußeren Beutel als Sauerstoffbarriere zusammen mit einem Desoxidationsmittel gebracht wurde.

9. Infusionszubereitung zur peripheren parenteralen Verabreichung nach Anspruch 1, die eine Hitzesterilisation durchläuft.

10. Verfahren zur Stabilisierung von Vitamin B1 in einer Infusionszubereitung zur peripheren parenteralen Verabreichung, das eine Infusion (A) umfasst, die Vitamin B1 und 80 bis 200 g/L Glucose enthält und eine Infusion (B), die 50 bis 150 g/L Aminosäure enthält, und auf einen pH-Wert von 6,5 bis 8 eingestellt ist, die separat in einen Behälter eingebracht werden, der unterteilt ist mit einem zu öffnenden Abtrennmittel, und wobei nach dem Mischen der Infusion (A) und der Infusion (B) die Mischung einen pH-Wert von 6 bis 7,5 und einen titrierbaren Säuregehalt von 5 bis 10 aufweist,
wobei die Infusion (A) kein Sulfit enthält, einen titrierbaren Säuregehalt von 1 oder weniger aufweist, auf einen pH-Wert von 3 bis 5 eingestellt ist, Carbonsäure und ihr Salz in einer Konzentration von 0 bis 5 mEq/L enthält und wobei alle weitere Elektrolyte, die in der Infusion (A) enthalten sind, starke Elektrolyte sind, und
die Infusion (A) eine Kaliumkonzentration von 10 bis 20 mEq/L und Infusion (B) eine Kaliumkonzentration von 20 bis 40 mEq/L aufweist.

## Revendications

1. Préparation de perfusion pour administration intraveineuse périphérique, comprenant une perfusion (A) contenant du glucose dans une concentration de 80 à 200 g/L et une perfusion (B) contenant un acide aminé dans une concentration de 50 à 150 g/L lesquelles sont placées séparément dans un récipient qui est divisé par des moyens de cloisonnement pouvant être ouverts ;
dans laquelle la perfusion (A) ne contient pas de sulfite, présente une acidité titrable inférieure ou égale à 1, contient de la vitamine B1, a un pH ajusté de 3 à 5, contient de l'acide carboxylique et son sel dans une concentration de 0 à 5 mEq/L, et dans laquelle d'autres électrolytes présents dans la perfusion (A) sont tous des électrolytes forts ;
la perfusion (B) a un pH ajusté de 6,5 à 8 ;
une fois la perfusion (A) et la perfusion (B) mélangées, le mélange à un pH de 6 à 7,5 et une acidité titrable de 5 à 10 ;
et la perfusion (A) a une concentration en potassium de 10 à 20 mEq/L et la perfusion (B) a une concentration en potassium de 20 à 40 mEq/L.

2. Préparation de perfusion pour administration intraveineuse périphérique selon la revendication 1, dans laquelle la perfusion (A) a une composition électrolytique de Ca²⁺ : de 2 à 10 mEq/L, Mg²⁺ : de 2 à 10 mEq/L, Cl⁻ : de 12 à 30 mEq/L, Zn : de 2 à 10 mmol/L ; la perfusion (B) a une composition électrolytique de Na⁺ : de 80 à 150 mEq/L, P : de 10 à 20 mmol/L ; et le rapport volumique (A:B) de la perfusion (A) à la perfusion (B) est de 1 à 4:1.

3. Préparation de perfusion pour administration intraveineuse périphérique selon la revendication 2, dans laquelle le calcium et le potassium se présentent respectivement sous forme de chlorure comme électrolytes forts ; et le chlorure de sodium n'est utilisé comme source d'alimentation en sodium ni dans l'une ni dans l'autre des perfusions (A) et (B).

4. Préparation de perfusion pour administration intraveineuse périphérique selon la revendication 1, dans laquelle la composition d'acide aminé de la perfusion (B) se présente de la façon suivante en termes d'acide aminé libre : L-leucine : de 10 à 20 (g/L), L-isoleucine : de 5 à 15 (g/L), L-valine : de 5 à 15 (g/L), L-lysine : de 5 à 15 (g/L), L-thréonine : de 2 à 10 (g/L), L-triptophane : de 0,5 à 5 (g/L), L-méthionine : de 1 à 8 (g/L), L-phénylalanine : de 3 à 15 (g/L), L-cystéine : de 0,1 à 3 (g/L), L-tyrosine : de 0,1 à 2 (g/L), L-arginine : de 5 à 15 (g/L), L-histidine : de 2 à 10 (g/L), L-alanine : de 5 à 15 (g/L), L-proline : de 2 à 10 (g/L), L-sérine : de 1 à 7 (g/L), glycine : de 2 à 10 (g/L), acide L-aspartique : de 0,2 à 3 (g/L), acide L-glutamique : de 0,2 à 3 (g/L).

5. Préparation de perfusion pour administration intraveineuse périphérique selon la revendication 4, dans laquelle la L-cystéine se présente sous forme de composé N-acétylé.

6. Préparation de perfusion pour administration intraveineuse périphérique selon la revendication 5, dans laquelle la perfusion (A) contient de la vitamine B1 dans une concentration de 1 à 10 mg/L en termes de thiamine.

7. Préparation de perfusion pour administration intraveineuse périphérique selon la revendication 1, dans laquelle le récipient est une poche à perfusion en plastique souple comportant au moins deux chambres qui sont séparées par un joint pouvant être aisément enlevé.

8. Préparation de perfusion pour administration intraveineuse périphérique selon la revendication 7, dans laquelle la poche à perfusion a été pliée au niveau de la partie du joint pouvant être aisément enlevé et a été placée dans une poche de conditionnement externe imperméable à l'oxygène, conjointement avec un désoxydant.

9. Préparation de perfusion pour administration intraveineuse périphérique selon la revendication 1, qui est soumise à une stérilisation par la chaleur.

10. Procédé permettant de stabiliser la vitamine B1 dans une préparation de perfusion pour administration intraveineuse périphérique qui comprend une perfusion (A) contenant de la vitamine B1 et de 80 à 200 g/L de glucose et une perfusion (B) contenant de 50 à 150 g/L d'acide aminé et dont le pH est ajusté de 6,5 à 8 lesquelles sont placées séparément dans un récipient qui est divisé par des moyens de cloisonnement pouvant être ouverts et dans laquelle une fois la perfusion (A) et la perfusion (B) mélangées, le mélange à un pH de 6 à 7,5 et une acidité titrable de 5 à 10,
dans lequel la perfusion (A) ne contient pas de sulfite, présente une acidité titrable inférieure ou égale à 1, a un pH ajusté de 3 à 5, contient de l'acide carboxylique et son sel dans une concentration de 0 à 5 mEq/L, et dans lequel d'autres électrolytes présents dans la perfusion (A) sont tous des électrolytes forts,
et la perfusion (A) a une concentration en potassium de 10 à 20 mEq/L et la perfusion (B) a une concentration en potassium de 20 à 40 mEq/L.
